Europäisches Patentamt

European Patent Office

Office européen des brevets

(11) Publication number: **0 654 260 A1**

# EUROPEAN PATENT APPLICATION

(21) Application number: **94117821.2**

(22) Date of filing: **11.11.94**

(51) Int. Cl.6: **A61K 7/46**, A61K 7/32

(30) Priority: **23.11.93 EP 93118791**

(43) Date of publication of application:
**24.05.95 Bulletin 95/21**

(84) Designated Contracting States:
**CH DE ES FR GB IT LI NL**

(71) Applicant: **GIVAUDAN-ROURE (INTERNATIONAL) S.A.**

**CH-1214 Vernier, Genève (CH)**

(72) Inventor: **Baydar, Ahmet Ennis**
**9, rue Yvon Villarceau**
**F-75116 Paris (FR)**
Inventor: **Charles, André Pascal**
**9, rue de Montmorency**
**F-95230 Soisy sous Montmorency (FR)**

(74) Representative: **Urech, Peter, Dr. et al**
**Grenzacherstrasse 124**
**Postfach 3255**
**CH-4002 Basel (CH)**

(54) **Odorant compositions.**

(57) Novel aqueous odorant compositions comprising a perfume concentrate, panthenol (ether) and a surfactant.

The present invention is concerned with novel odorant compositions. More particularly, the invention is concerned with novel odorant compositions with prolonged diffusion after the application on the skin.

It is well known that mixtures of perfume materials when deposited on the skin lose intensity and may change character with time, mainly due to factors such as differential evaporation and skin penetration.

Many attempts have been made to minimize these drawbacks, but so far without notable success. Particularly, efforts have been made to prolong the diffusion, as well as to improve other characteristics of perfume materials, by e.g. increasing the perfume concentrate concentration or by using additives such as silicones, glycerol, polyethylene glycols and so on. Such perfume compositions, however, have never been really successful as the results obtained were only marginal.

Recently, odorant compositions have been described, which are devoid of the drawbacks outlined above, see EP 558 721. These compositions, though, all contain substantial amounts of ethanol.

The present invention now provides such prolonged diffusivity of non - alcoholic, namely aqueous perfume solutions.

Surprisingly, it has been found that the addition of panthenol (or an alkyl ether thereof) significantly improves and particularly prolongs diffusion of perfume materials from the skin, without notably modifying the olfactory note of the product. The present invention thus concerns odorant compositions comprising from about 0.1% to about 30% of perfume concentrate, from about 0.1% to about 10% of panthenol or an alkyl, e.g. ethyl ether thereof, from about 0.1% to about 50% of a surfactant and from about 10% to about 99% of water.

The term "odorant composition", as used in connection with the present invention, means solutions of perfume materials and stands for well known commercial products such as e.g. Deo-Cologne, Eau de Cologne, Eau de Toilette, Perfume Extrait and so on, though necessitating no alcohol.

The term "perfume concentrate" stands for the required mixture of any perfume materials of synthetic and/or natural origin.

The term "panthenol" as used in connection with the present invention stands for DL-panthenol or for D-panthenol.

The nature of the surfactant, which operates as a solubilizer, is by no means critical and encompasses thus anionic, cationic and amphotheric surfactants. Its concentration is conveniently between ca. 0.1 and ca. 50%.

A representative list of such surfactants can be found in Cosmetics & Toiletries vol. 108, 1993, pages 71 seq.

Preferred surfactants are of the non-ionic type, in particular of the polyoxyethylene type.

Particularly preferred surfactants are LRI (CFTA Name: PPG 26 - Buteth 26 - PEG 40 - Hydrogenated Castor oil), CREMOPHOR RH 40 (Polyoxyethylene glycerol trihydroxystearate 40 DAC), TWEEN 20 (Polyoxyethylene 20 sorbitan monolaurate).

Further preferred surfactants are:

CREMOPHOR RH 60 (PEG-60 hydrogenated castor oil); PEG-60 hydrogenated Castor Oil is a polyethylene glycol derivative of hydrogenated castor oil with an average of 60 moles of ethylene oxide,

TWEEN 80 (Polysorbate 80, i.e. a mixture of oleate esters of sorbitol and sorbitol anhydrides, consisting predominantly of the monoester, condensed with approximately 20 moles of ethylene oxide),

TRITON X 100 (Octoxynol-9),

LAMACIT 877 (Nonoxynol-14),

TRITON X 102 (Octoxynol-13).

The percentages referred to in the scope of the present invention are on a weight by weight basis.

The amount of perfume concentrate present depends upon the respective odorant composition and can be easily determined by the skilled artisan. The amount of panthenol (or panthenol ether) can also vary within a range of from about 0.1 to about 10%, but lies preferably in the range of from about 0.5 to about 5%. By way of examples, in the following odorant compositions the respective ingredients may be present in the following approximate amounts:

| a) Deo-Cologne: | |
| --- | --- |
| Perfume concentrate | 0.1-3% |
| Panthenol (ether) | 0.1-1% |
| Water | q.s. |

| b) Eau de Cologne: | |
|---|---|
| Perfume concentrate | 2-7% |
| Panthenol (ether) | 0.5-5% |
| Water | q.s. |

| c) Eau de Toilette: | |
|---|---|
| Perfume concentrate | 5-20% |
| Panthenol (ether) | 0.5-5% |
| Water | q.s. |

| d) Perfume Extrait: | |
|---|---|
| Perfume concentrate | 15-25% |
| Panthenol (ether) | 0.5-5% |
| Water | q.s. |

The ratio of perfume concentrate to panthenol (ether) lies conveniently between ca. 10 : 5 to ca. 10 : 0,5.

The ratio of perfume concentrate to surfactant lies, as will be shown below, conveniently between ca. 1 to ca. 3.

Besides the ingredients mentioned before, the odorant compositions according to the present invention can also contain additives which are well known in the art. By way of example the following can be mentioned: colorants, UV-absorbants, antioxidants, preservatives, emollients, natural herbal extracts, germicides, deodorants and so on. If such additions are present their amount lies preferably between about 0.05 and about 2%.

Furthermore, the odorant compositions can also contain vitamins, such as e.g. vitamin E, preferably in the form of the acetate, vitamin A, preferably in the form the acetate or any other common ester, vitamin C, preferably in the form of the palmitate, and so on.

The following examples are illustrative of the present invention and are by no means intended to limit the scope of the present invention.

Example 1

In a manner known per se a Deo-Cologne of the following composition has been prepared:

| Perfume concentrate (100%) | 1% |
|---|---|
| D-Panthenol | 0.2% |
| Solubilizer, e.g. LRI | 1,5% |
| Water | q.s. |

Example 2

In a manner known per se an Eau de Cologne of the following composition has been prepared:

| Perfume concentrate (100%) | 5% |
|---|---|
| D-Panthenol | 1% |
| Solubilizer, e.g. LRI | 7,5% |
| Water | q.s. |

Example 3

In a manner known per se an Eau de Toilette of the following composition has been prepared:

| | |
|---|---|
| Perfume concentrate (100%) | 10% |
| D-Panthenol | 2% |
| Solubilizer, e.g. LRI | 15% |
| Water | q.s. |

The very substantial and surprising effect of all the novel compositions, as compared with identical aqueous solutions containing no panthenol (ether), was first of all determined by half-quantitative sniffing tests on the forearm of a number of panellists, wherby samples of 3 microliters of the compositions/cm$^2$ were applied.

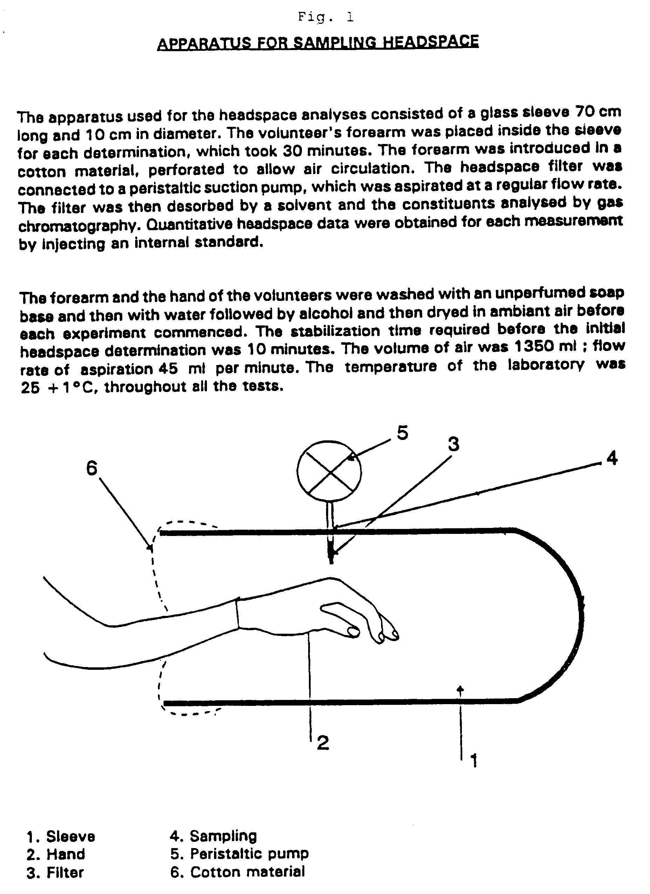

A quantitative determination followed, using headspace analysis on the skin, carried out with the apparatus of Figure 1. Measurements were carried out with a number of completely different odorants, representing constituents of a perfume formula. A typical set of data obtained is given below. The data represent the amount of material detected in ng/L over the skin of each perfume raw material as applied. The headspace determinations were carried out at T = 0 and T = 4 hrs after application of the perfume composition on the skin. The perfume composition was composed of

| | |
|---|---|
| odorant | 10% |
| surfactant, i.e. LRI | 15% |
| d-panthenol | 2% |
| water | q.s. |

Comparing the results, the amount of diffused perfumery material applied via panthenol was in the large majority of cases several times, e.g. 2 to 10 times the amount of the perfume applied without using panthenol (Table 1).

The quantity of the odour liberated into the air was in each case determined as follows:

A defined volume of the fragrances was aspirated at a regular flow through the filter containing an adsorbant shown in Figure 1. After desorption from the adsorbant by a solvent, the constituents were analysed by gas chromatography on polar and apolar capillary columns with an internal standard to determine the concentration of each fragrance molecule in the air stream.

Each headspace experiment was also confirmed by an olfactive evaluation test carried out by 13 to 18 evaluators.

The other surfactants used in Examples 1 to 3 lead to analogous results.

## QUANTITY IN ng/l IN HEADSPACE

| | T = 0hr | | T = 4hrs | |
|---|---|---|---|---|
| Components | Without Panthenol | With Panthenol | Without Panthenol | With Panthenol |
| alpha-pinene | 6244 | 85209 | 9 | 58 |
| limonene | 5536 | 89912 | 21 | 151 |
| meta or para-cresol | 4186 | 49048 | 60 | 433 |
| dimetol | 2606 | 52451 | 12 | 189 |
| linalool | 2853 | 25588 | 48 | 331 |
| linalyl acetate | 2773 | 24642 | 23 | 199 |
| bornyl acetate | 2881 | 24867 | 25 | 232 |
| aldehyde C 11 | 861 | 7163 | 6 | 167 |
| estragol | 4102 | 44449 | 42 | 401 |
| benzyl acetate | 1618 | 17675 | 21 | 167 |
| citronellol | 363 | 2961 | 12 | 80 |
| nerol | 533 | 4154 | 17 | 118 |
| phenylethylic alcohol | 1083 | 11368 | 42 | 310 |
| cyclamen aldehyde | 348 | 2363 | 17 | 173 |
| diphenyl oxide | 397 | 2618 | 42 | 333 |
| isoamyl salicylate | 842 | 6751 | 22 | 148 |
| amyl salicylate | 104 | 760 | 13 | 94 |
| eugenol | 136 | 991 | 34 | 215 |
| heliotropine | 132 | 1203 | 32 | 199 |

5

| | | | | |
|---|---|---|---|---|
| isoeugenol | 65 | 477 | 25 | 137 |
| diethyl phthalate | 137 | 1269 | 12 | 77 |
| coumarine | 37 | 181 | 21 | 97 |
| vanilline | 36 | 148 | 23 | 91 |
| benzyl salicylate | traces | 54 | traces | traces |
| Jasmal | 74 | 392 | 39 | 187 |
| Rosone | 35 | 142 | 23 | 85 |
| Hedione | 58 | 502 | 12 | 80 |
| Galaxolide | 23 | 115 | 6 | 23 |
| Fixolide | traces | traces | traces | 5 |
| Lyral | 45 | 151 | 20 | 64 |
| Total | 38108 | 457604 | 679 | 4844 |

## Claims

1. Odorant composition comprising from about 0.1% to about 30% of perfume concentrate, from about 0.1% to about 10% of panthenol or an alkyl, e.g. ethyl, ether thereof, from about 0.1% to about 50% of a surfactant and from about 10% to about 99% of water.

2. Odorant composition according to Claim 1, in the form of a Deo-Cologne, comprising from about 0.1 to about 3% of perfume concentrate and from about 0.1 to about 1% of panthenol or an alkyl ether thereof.

3. Odorant composition according to Claim 1, in the form of an Eau de Cologne, comprising from about 2 to about 7% of perfume concentrate and from about 0.5 to about 5% of panthenol or an alkyl ether thereof.

4. Odorant composition according to Claim 1, in the form of an Eau de Toilette, comprising from about 5 to about 20% of perfume concentrate and from about 0.5 to about 5% of panthenol or an alkyl ether thereof.

5. Odorant composition according to Claim 1, in the form of a Perfume Extrait, comprising from about 15 to about 25% of perfume concentrate and from about 0.5 to about 5% of panthenol or an alkyl ether thereof.

6. The use of panthenol or an alkyl ether thereof in aqueous odorant compositions containing a surfactant for prolonging diffusion after the application on the skin.

## Fig. 1

### APPARATUS FOR SAMPLING HEADSPACE

The apparatus used for the headspace analyses consisted of a glass sleeve 70 cm long and 10 cm in diameter. The volunteer's forearm was placed inside the sleeve for each determination, which took 30 minutes. The forearm was introduced in a cotton material, perforated to allow air circulation. The headspace filter was connected to a peristaltic suction pump, which was aspirated at a regular flow rate. The filter was then desorbed by a solvent and the constituents analysed by gas chromatography. Quantitative headspace data were obtained for each measurement by injecting an internal standard.

The forearm and the hand of the volunteers were washed with an unperfumed soap base and then with water followed by alcohol and then dryed in ambient air before each experiment commenced. The stabilization time required before the initial headspace determination was 10 minutes. The volume of air was 1350 ml ; flow rate of aspiration 45 ml per minute. The temperature of the laboratory was 25 + 1°C, throughout all the tests.

1. Sleeve
2. Hand
3. Filter
4. Sampling
5. Peristaltic pump
6. Cotton material

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | FR-A-2 213 051 (LIODES ANSTALT)<br>* the whole document *<br>--- | 1-6 | A61K7/46<br>A61K7/32 |
| A | WO-A-93 05761 (GIVAUDAN-ROURE)<br>* the whole document *<br>--- | 1-6 | |
| A | EP-A-0 261 351 (ROURE BERTRAND DUPONT S. A.)<br>* example k *<br>--- | 1-6 | |
| A | JANISTIJN 'Handbuch der Kosmetika und Riechstoffe, vol 3 : die Körperpflegemittel, 2 edition'<br>1973 , HUTHIG VERLAG , HEIDELBERG, DE<br>* page 467, " Gesichtwasser, reizlindernd " *<br>----- | 1-6 | |
| | | | TECHNICAL FIELDS SEARCHED (Int.Cl.6)<br><br>A61K |

The present search report has been drawn up for all claims

| Place of search | Date of completion of the search | Examiner |
|---|---|---|
| THE HAGUE | 15 February 1995 | Fischer, J.P. |

EPO FORM 1503 03.82 (P04C01)